# EUROPEAN PATENT APPLICATION

(11) **EP 4 837 627 A1**
(43) Date of publication of application: **11.11.2026**
(21) Application number: 25741367.4
(22) Date of filing: 06.01.2025
(51) Int. Cl.: A61N 7/00

(54) **ULTRASOUND THERAPY SYSTEM AND USE THEREOF**

(30) Priority: 18.01.2024 WO PCT/CN2024/073058
(71) Applicant: Shanghai A & S Science Technology Development Co., Ltd., Shanghai 201201 (CN)
(72) Inventor: HE, Ye, Shanghai 201201 (CN)
(74) Representative: dompatent
(86) International application number: PCT/CN2025/070746
(87) International publication number: WO 2025/152804

(57) **Abstract**

Disclosed in the present invention are an ultrasound therapy system and use thereof. The ultrasound therapy system comprises a main unit and a therapy head. The main unit comprises a signal module, a power supply module, an output module, and a matching module. The therapy head comprises a piezoelectric module and an amplitude transformer module. The length of the piezoelectric module and the length of the amplitude transformer module are half of the wavelength. The frequency range output by the ultrasound therapy system conforms to the frequency range of ultrasonic waves output by bats. According to the present invention, low-frequency and low-intensity ultrasound acts on the immune system of an organism, and specific immune environments such as interferon a high expression, immune strong response, autophagy enhancement, and inflammation level down-regulation are formed in the organism. Therefore, the ultrasound therapy system has the advantages of resisting tumors and viruses, preventing and ameliorating inflammatory aging, prolonging the life span, and treating autoimmune diseases.

## Description

The present application claims priority to Chinese patent application PCT/CN2024/073058, filed on January 18, 2024. The contents of the above Chinese patent application are incorporated herein by reference in their entireties.

### TECHNICAL FIELD

The present disclosure relates to the field of medical devices, and particularly to an ultrasound therapy system and the use thereof.

### BACKGROUND

Bats carry hundreds of highly pathogenic viruses, such as SARS virus, rabies virus, Ebola virus, and COVID-19. However, the viral load carried by bats is relatively low and does not cause corresponding symptoms, allowing them to coexist with these viruses. Even more astonishing is the lifespan of bats, which is much longer than that of terrestrial mammals of similar body size. One reason for the longevity of bats is their ability to regulate inflammation, and the underlying mechanisms may provide novel approaches for delaying aging.

The question arises as to what biological mechanisms make bats so unique that they can tolerate a wide range of viruses while ensuring their own safety. Innate immune responses are capable of sensing cytoplasmic DNA and exogenous RNA, resulting in the upregulation of interferon genes and the production of other inflammatory cytokines, such as TNF-α. Through evolutionary adaptation, bats have attenuated their sensing of cytoplasmic DNA by losing PYHIN genes and acquiring mutations in regulatory sites of STING.

In bats, the expression levels of TNF-α and other inflammatory cytokines are reduced, whereas anti-inflammatory cytokines, such as IL-10, are upregulated. In addition, activation of the NLRP3 inflammasome is suppressed in bats, leading to reduced production of IL-1β and IL-18, while RNA sensing is enhanced in bats. Furthermore, bats constitutively express TNF-α and exhibit enhanced expression of interferon genes, while also possessing stronger autophagy capacity. In summary, these adaptive changes in bats enable them to tolerate viruses while suppressing inflammatory responses.

Alterations in immunity, leading to increased viral tolerance and reduced inflammation, constitute a basis for the longevity of bats. These changes are associated with a reduction in inflammation-related diseases, such as atherosclerosis, arthritis, neurodegenerative disorders, and cancer. Consequently, immune changes extend the lifespan of bats.

Studies of bat biology have revealed that bats are among the very few mammals that utilize ultrasound for navigation and predation. Another mammal known to utilize ultrasound for navigation is the naked mole-rat, which is likewise capable of coexisting with a large number of viruses and exhibits exceptional longevity. Therefore, the ability of bats and naked mole-rats to tolerate viruses and suppress inflammatory responses, as well as their longevity, may be associated with ultrasound.

### SUMMARY

In order to solve the technical problem in the prior art of lacking a device capable of effectively stimulating immune responses of the body, exerting anti-tumor and anti-viral effects, preventing and ameliorating inflammatory aging, extending lifespan, and treating autoimmune diseases, the present disclosure provides an ultrasound therapy system and the use thereof.

To address the above technical problem, the present disclosure provides the following technical solution: an ultrasound therapy system, comprising a main unit and a treatment head;
the main unit comprises the following modules:
   (1) a signal module, configured to generate a fundamental frequency signal of ultrasound;
   (2) a power supply module, configured to provide a constant DC voltage;
   (3) an output module, configured to amplify the signal output;
   (4) a matching module, configured to match the output signal with a piezoelectric module of the treatment head to achieve consistency in voltage and current phases;
the treatment head comprises the following modules:
   (i) a piezoelectric module, configured to convert electrical energy into mechanical energy;
   (ii) an amplitude transformer module, configured to output the vibration generated by the piezoelectric module;
the lengths of the piezoelectric module and the amplitude transformer module are each half of a wavelength;
the frequency range output by the ultrasound therapy system conforms to the frequency range of ultrasonic waves output by bats.

In a specific embodiment of the present disclosure, the main unit further comprises: (5) a display module, configured to set parameters for ultrasound therapy, including frequency, sound intensity, duration, and/or duty cycle, and to provide functions for initiating and terminating ultrasound therapy;
the treatment head further comprises: (iii) a packaging module, configured to encapsulate and secure the piezoelectric module and the amplitude transformer module therein; and/or
the ultrasound therapy system further comprises a positioning module for locating a treatment area.

In a specific embodiment of the present disclosure, the ultrasonic waves output by the ultrasound therapy system are in the form of a sine wave; the frequency output by the ultrasound therapy system ranges from 20 to 50 kHz, with an output intensity ranging from 0.1 to 1.6 W/cm², a pulse width ranging from 10 to 1000 ms, and a period ranging from 20 to 2000 ms, corresponding to a duty cycle of 1/2 to 1/200.

In a specific embodiment of the present disclosure, the frequency output by the ultrasound therapy system ranges from 30 to 40 kHz, the output intensity ranges from 0.15 to 0.25 W/cm², the pulse width ranges from 50 to 200 ms, and the period ranges from 300 to 500 ms.

In a specific embodiment of the present disclosure, the frequency output by the ultrasound therapy system ranges from 30 to 50 kHz, the output intensity ranges from 0.15 to 0.25 W/cm², the pulse width ranges from 50 to 200 ms, and the period ranges from 300 to 400 ms.

In a specific embodiment of the present disclosure, the ultrasonic waves output by the ultrasound therapy system are further processed using a smooth-transition algorithm.

In a specific embodiment of the present disclosure, the ultrasound therapy system satisfies one or more of the following conditions:
the display module comprises a liquid crystal screen;
the signal module generates the fundamental frequency signal of ultrasound by means of DDS;
the output module amplifies the signal output by means of a MOS transistor;
the piezoelectric module comprises one or more piezoelectric ceramic wafers; and
the positioning module is a B-mode ultrasound probe, preferably a miniature B-mode ultrasound probe.

In a specific embodiment of the present disclosure, the positioning module is fixed on one side of the treatment head, both being located on the same plane and forming an angle of 0° to 90°, such that the acoustic pathway of therapeutic ultrasound is displayed on the B-mode ultrasound image.

In a specific embodiment of the present disclosure, the angle is 10° to 80°, preferably 30° to 60°.

In a specific embodiment of the present disclosure, the piezoelectric module satisfies one or more of the following conditions:
the piezoelectric module comprises four piezoelectric ceramic wafers;
the piezoelectric ceramic wafers are P42 piezoelectric ceramics; and
the piezoelectric ceramic wafers are arranged with alternating positive and negative electrodes.

In a specific embodiment of the present disclosure, the packaging module comprises: a front cover plate, a rear cover plate, a housing, and a fastener, wherein the density of the material of the rear cover plate is greater than that of the front cover plate.

In a specific embodiment of the present disclosure, the packaging module satisfies one or more of the following conditions:
the front cover plate is made of aluminum alloy;
the rear cover plate is made of stainless steel;
the housing is adapted for handheld operation, preferably in a cylindrical shape; and
the fastener is a set screw configured to securely connect the respective modules; the set screw is preferably provided with a preload.

In a specific embodiment of the present disclosure, the amplitude transformer module is in a rectangular shape, a circular shape, a spherical surface shape, a honeycomb shape, a needle shape, or a ball head shape.

In a specific embodiment of the present disclosure, the amplitude transformer module satisfies one or more of the following conditions:
the output surface of the amplitude transformer module is wrapped with a silicone rubber film;
the transition between different diameters of the amplitude transformer module is smoothed by a tangent circular surface; and
the amplitude transformer module further comprises a flange for mounting the housing; preferably, the flange is positioned at a node.

In a specific embodiment of the present disclosure, the ultrasound therapy system is used for regulating the following changes in the body:
(1) increasing the expression of interferons and/or interleukins; the interferon comprises IFN-α, IFN-β, and IFN-γ, and the interleukin comprises IL-10 and IL-15;
(2) promoting the proliferation of CD4+ T cells and/or CD40+ immune cells, and/or reducing the ratio of CD8/CD4.

To address the above technical problem, the present disclosure provides the following technical solution: a method of stimulating an immune response in a body using the ultrasound therapy system described herein, wherein ultrasonic stimulation is administered to a target site, preferably an immune organ such as the spleen or brain, comprising the following steps:
(1) bringing the ultrasound therapy system close to or in contact with the target site;
(2) adjusting parameters such that the ultrasound therapy system generates and emits ultrasonic waves;
(3) adjusting the propagation direction, output frequency, intensity, pulse width, period, and output duration of the ultrasonic waves according to the location and area of the target site.

In a specific embodiment of the present disclosure, the stimulation of the immune response in the body comprises anti-tumor therapy, anti-viral therapy, prevention and amelioration of inflammatory aging, lifespan extension, and/or treatment of autoimmune diseases.

In a specific embodiment of the present disclosure, the frequency output by the ultrasound therapy system ranges from 20 to 50 kHz, with an output intensity ranging from 0.1 to 1.6 W/cm², a pulse width ranging from 10 to 1000 ms, and a period ranging from 20 to 2000 ms, corresponding to a duty cycle of 1/2 to 1/200; and the duration of ultrasonic stimulation ranges from 60 to 600 s.

In a specific embodiment of the present disclosure, the frequency output by the ultrasound therapy system ranges from 30 to 40 kHz, with an output intensity ranging from 0.15 to 0.25 W/cm², a pulse width ranging from 50 to 200 ms, and a period ranging from 300 to 500 ms, corresponding to a duty cycle of 1/2 to 1/200; and the duration of ultrasonic stimulation ranges from 120 to 240 s.

In a specific embodiment of the present disclosure, the frequency output by the ultrasound therapy system ranges from 30 to 50 kHz, with an output intensity ranging from 0.15 to 0.25 W/cm², a pulse width ranging from 50 to 200 ms, and a period ranging from 300 to 400 ms, corresponding to a duty cycle of 1/2 to 1/200; and the duration of ultrasonic stimulation ranges from 120 to 240 s.

In a specific embodiment of the present disclosure, the frequency output by the ultrasound therapy system is 33 kHz, the output intensity is 0.2 W/cm², the pulse width is 100 ms, the period is 400 ms, and the duration of ultrasonic stimulation is 180 s.

In a specific embodiment of the present disclosure, the frequency output by the ultrasound therapy system is 50 kHz, the output intensity is 0.2 W/cm², the pulse width is 100 ms, the period is 400 ms, and the duration of ultrasonic stimulation is 180 s.

In a specific embodiment of the present disclosure, ultrasonic stimulation is administered to the target site when the body has already developed a tumor, has already been infected with a virus, has already developed inflammation, has already aged, or has already developed an autoimmune disease.

To address the above technical problem, the present disclosure provides the following technical solution: use of the ultrasound therapy system described herein in the manufacture of a device for stimulating an immune response in a body.

In a specific embodiment of the present disclosure, the stimulation of the immune response in the body comprises anti-tumor therapy, anti-viral therapy, prevention and amelioration of inflammatory aging, lifespan extension, and/or treatment of autoimmune diseases.

To address the above technical problem, the present disclosure provides the following technical solution: the ultrasound therapy system described herein for use in stimulating an immune response in a body.

In a specific embodiment of the present disclosure, the stimulation of the immune response in the body comprises anti-tumor therapy, anti-viral therapy, prevention and amelioration of inflammatory aging, lifespan extension, and/or treatment of autoimmune diseases.

On the basis of common knowledge in the art, the above preferred conditions may be arbitrarily combined to obtain various preferred examples of the present disclosure.

The reagents and raw materials used in the present disclosure are all commercially available.

The positive and progressive effects of the present disclosure are as follows:
In the present disclosure, low-frequency and low-intensity ultrasound applied to the immune system can create a specific immune environment in the body characterized by high expression of interferon-α, strong immune responses, enhanced autophagy, and reduced inflammation. Therefore, anti-tumor and anti-viral effects, prevention and amelioration of inflammatory aging, lifespan extension, and treatment of autoimmune diseases can be achieved.

### BRIEF DESCRIPTION OF THE DRAWINGS

FIG. 1 is a schematic diagram of certain modules of the main unit.
FIG. 2 is a schematic diagram of piezoelectric ceramic wafers arranged with alternating positive and negative electrodes.
FIG. 3 is a schematic diagram of a horn tool head having a rectangular or spherical output surface.
FIG. 4 is a schematic diagram of a horn tool head having a rectangular output surface.
FIG. 5 is a schematic diagram of a horn tool head having a spherical output surface.
FIG. 6 is a schematic diagram of the mounting structure of the housing of the horn tool head.
FIG. 7 shows the structure of the treatment head.
FIG. 8 shows the structure of the piezoelectric ceramic.
FIG. 9 shows the structure of the horn tool head.
FIG. 10 is a schematic diagram of a sine wave.
FIG. 11 is a schematic diagram showing sine wave modulation.
FIG. 12 is a schematic diagram showing the output ultrasonic waves processed using a smooth-transition algorithm.
FIG. 13 shows the mounting configuration of the positioning module and the treatment head.
FIG. 14 is a schematic diagram of the therapeutic ultrasound and the target organ on the image from the positioning module.
FIG. 15 shows tumor size and tumor growth curves.
FIG. 16 shows histopathological analysis of tumors.
FIG. 17 shows statistical analysis of cytokines in the blood of mice.
FIG. 18 shows statistical analysis of immune cells in the blood of mice.
FIG. 19 shows statistical analysis of cytokines in the blood of rats.

### DETAILED DESCRIPTION OF THE PREFERRED EMBODIMENT

The following examples further illustrate the present disclosure, but the present disclosure is not limited thereto.

The technical solution proposed by the present disclosure is to stimulate immune responses in a body through the use of ultrasound.

Ultrasonic stimulation alters the immune environment within the body and induces a series of immune responses, including high expression of interferon-α, enhanced viral tolerance, and improved control of inflammation. Based on the ultrasonic emissions of bats and other related physiological characteristics, the inventors developed a dedicated ultrasound therapy system.

The main parameters of the instrument are as follows:
- Frequency: 20 kHz to 50 kHz
- Sound intensity: 0.1 to 1.6 W/cm²

The key evaluation indicators are as follows:
- IFN-α, IFN-β, IFN-γ, CD4+ T cells, CD8+ T cells, TNF-α, IL-10, IL-1β, IL-15, and IL-18.

The primary immunological effects produced by the instrument may be applied to the following aspects:
- Anti-tumor therapy
- Anti-viral therapy
- Prevention and amelioration of inflammatory aging and lifespan extension
- Treatment of autoimmune diseases

The inventors designed a series of animal experiments to evaluate the immunological effects induced by ultrasonic stimulation.

Experiment 1: A mouse liver tumor model was established. Following more than one year of animal experiments and repeated optimization of technical parameters, the treatment group, compared to the control group, exhibited a 42% increase in IFN-α, a 35% increase in IFN-β, a 34% increase in IFN-γ, a 72% increase in CD4+ T cells, and a 42% increase in IL-15, while the growth of tumor cells was also inhibited. These experiments demonstrated that ultrasonic stimulation of the immune system can induce interferon production and increase other beneficial immune factors and immune cells, thereby inhibiting the growth of tumor cells.

Experiment 2: A normal mouse model was established. Following a series of animal experiments, the treatment group, compared to the control group, exhibited reduced expression of TNF-α, increased levels of the anti-inflammatory cytokine IL-10, and decreased levels of the pro-inflammatory cytokines IL-1β and IL-18. These changes may contribute to inflammation control, delayed aging, and treatment of autoimmune diseases.

Experiment 3: A model of 12-month-old rats was established. Following animal experiments, the treatment group exhibited a 68% increase in the anti-inflammatory cytokine IL-10 compared to the control group. These changes may contribute to inflammation control, delayed aging, and treatment of autoimmune diseases.

The present disclosure is further described below by way of examples, but is not thereby limited to the scope of the described examples. Experimental methods without specific conditions in the following examples are performed in accordance with conventional methods and conditions, or in accordance with the instructions provided by the respective manufacturers.

### Example 1: Ultrasound therapy system

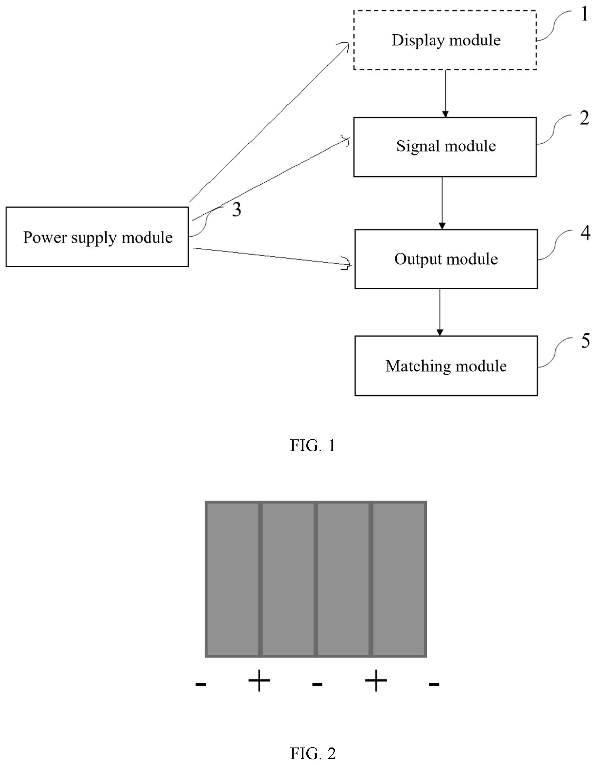

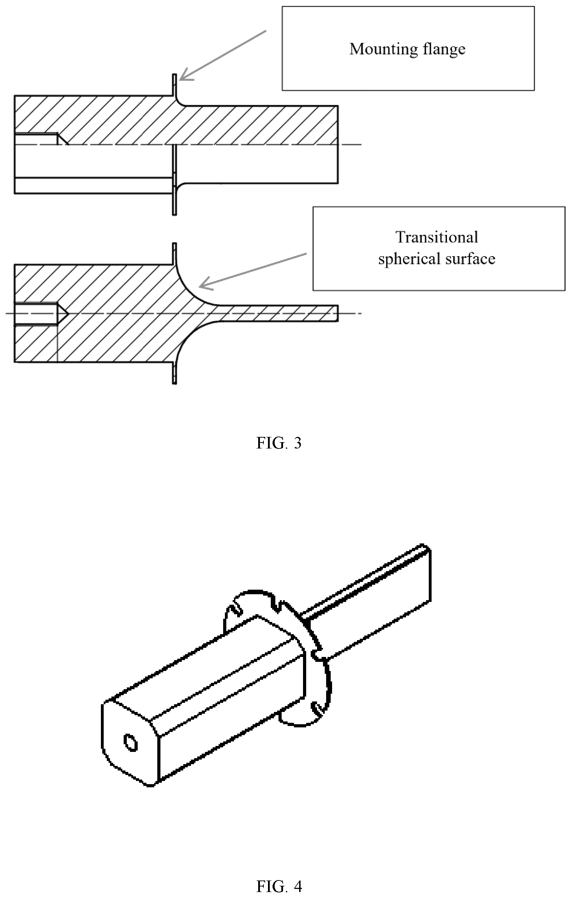

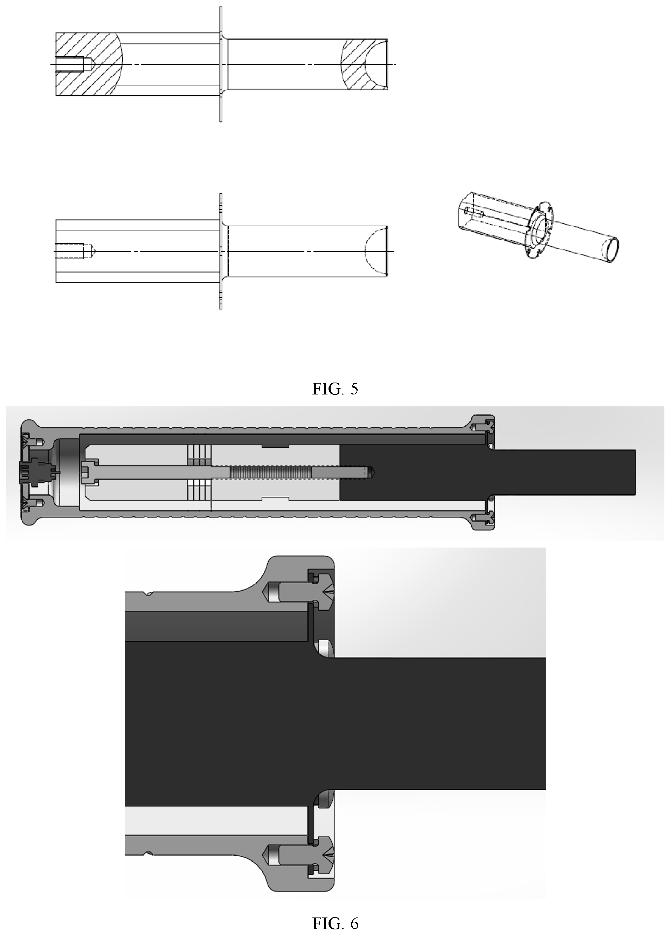

The ultrasound therapy system of the present disclosure comprises a main unit and a treatment head.
- The main unit comprises the following modules (as shown in FIG. 1):
   Display module: The display module mainly comprises a liquid crystal display (LCD), through which human-machine interaction is realized. Parameters for ultrasonic therapy, including frequency, sound intensity, duration, and duty cycle may be set via the LCD. The display module also provides functions for initiating and terminating ultrasonic therapy.
   Signal module: The signal module is configured to generate a fundamental frequency signal of ultrasound by means of DDS. A control unit generates an electrical signal for driving the ultrasound according to parameters including frequency, sound intensity, duration, and duty cycle.
   Power supply module: The power supply module is primarily configured to convert AC power into DC voltage and provide a constant DC voltage for each component.
   Output module: The output module is configured to amplify the signal output by means of a MOS transistor.
   Matching module: The matching module is configured to match the output electrical signal with the transducer to achieve consistency in voltage and current phases.
- The treatment head primarily generates a vibration through a piezoelectric transducer and outputs the vibration through a tool head. Both the piezoelectric transducer and the tool head are designed to be half the wavelength. The treatment head comprises the following modules:
   Piezoelectric ceramics: Piezoelectric ceramic wafers are components that convert electrical energy into mechanical energy and serve as the core components of the ultrasound therapy system. In the present device, four annular piezoelectric ceramic wafers made of P42 are employed. P42 piezoelectric ceramics exhibit high acoustic-to-electric conversion efficiency and are suitable for immunotherapy. The use of four annular piezoelectric ceramic wafers is determined based on the required acoustic power. If fewer wafers are used, the generated power may be insufficient to achieve the desired immunological effects, while increasing the thickness of individual wafers will increase manufacturing difficulty, which is not conducive to immunotherapy. The piezoelectric wafers have positive and negative electrodes. During assembly, the four wafers must be arranged with alternating positive and negative electrodes, as shown in FIG. 2.
   Horn tool head: Different horn geometries may be designed according to therapeutic requirements. For example, a rectangular horn may be designed for spleen treatment. Alternatively, a spherical horn may be designed to provide focusing characteristics to the transducer, as shown in FIG. 3. A schematic diagram of a horn tool head having a rectangular output surface is shown in FIG. 4. A schematic diagram of a horn tool head having a spherical output surface is shown in FIG. 5.

The horn tool head may be replaceable to accommodate different output waveform requirements. For example, the horn may also be designed in a honeycomb shape, a needle shape, a ball head shape, or other suitable geometries. Note that the horn design must meet the theoretical half-wavelength requirements.

In conventional horn tool heads, the output surface is made of metal that comes into direct contact with the skin, which is not conducive to immunotherapy. In the present design, a silicone rubber film is provided on the output surface, which can avoid direct metal-to-skin contact, serve as an ultrasound coupling medium, and prevent the thermal effects of ultrasound from being directly transferred to human skin.

The transition between different diameters of the horn tool head is smoothed by a tangent circular surface, thereby improving ultrasonic transmission efficiency on the horn.

The horn tool head further comprises a flange for mounting the housing. The flange is positioned at a node, thereby minimizing the effect on the vibration of the transducer, as shown in FIG. 6.

Front cover plate: The front cover plate is made of aluminum alloy.

Rear cover plate: The rear cover plate is made of stainless steel.

Housing: The housing is designed in a cylindrical shape to facilitate handheld operation.

Set screw: The set screw is configured to securely connect the respective modules. The set screw must be provided with a preload, which must be maintained within an appropriate range. Double-stacked self-locking washers or disc washers should be installed. C-shaped washers are generally not preferred to prevent loosening during use. A torque wrench is used when installing the screw, ensuring that the applied torque corresponds to the preload range.

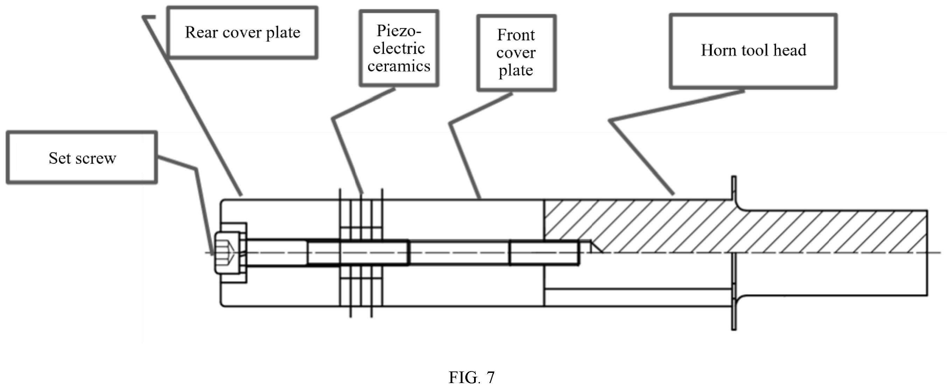

The treatment head of the present disclosure (structure shown in FIG. 7) generates ultrasonic waves when electrical power is applied to the piezoelectric ceramics. Since the density of stainless steel in the rear cover plate is high (approximately 7.9 g/cm³) while the density of aluminum alloy in the front cover plate is low (approximately 2.8 g/cm³), the forward displacement amplitude of the emitted ultrasonic waves is increased. The horn tool head positioned at the front further increases the amplitude of the emitted ultrasonic waves, enabling the treatment head of the present disclosure to provide high output efficiency and concentrated ultrasound energy. Furthermore, the dimensions of the horn tool head are customized according to the shape of the treatment site, enabling the entire treatment head to satisfy the requirements of immunotherapy.

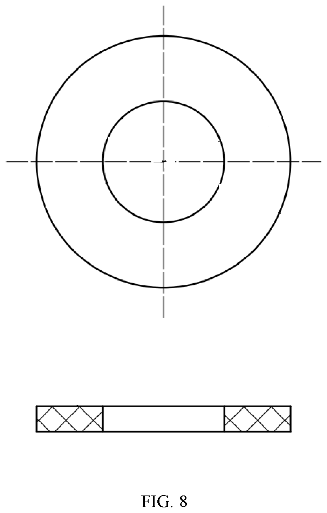

Piezoelectric ceramics (structure shown in FIG. 8) exhibit dielectric and elastic properties in addition to piezoelectric properties, and have been widely applied in medical imaging, acoustic sensors, acoustic transducers, ultrasonic motors, and related fields. Piezoelectric ceramics are manufactured by utilizing the piezoelectric effect, in which mechanical stress causes a relative displacement between positive and negative charge centers within the material, resulting in polarization and the appearance of bound charges of opposite signs on opposing surfaces, thereby providing high sensitivity.

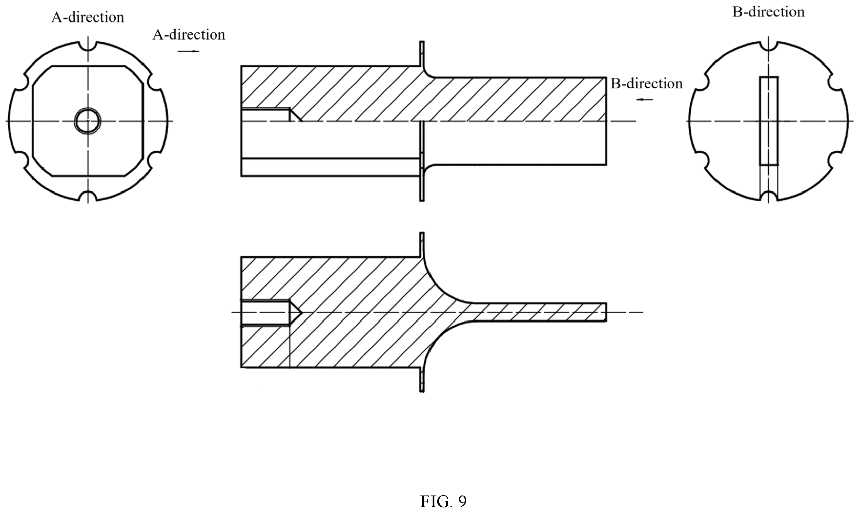

The output surface of the horn tool head (structure shown in FIG. 9) is configured as an elongated rectangular shape. The dimensions are selected according to the shape of the treatment site, enabling the entire treatment head to satisfy the requirements of immunotherapy.

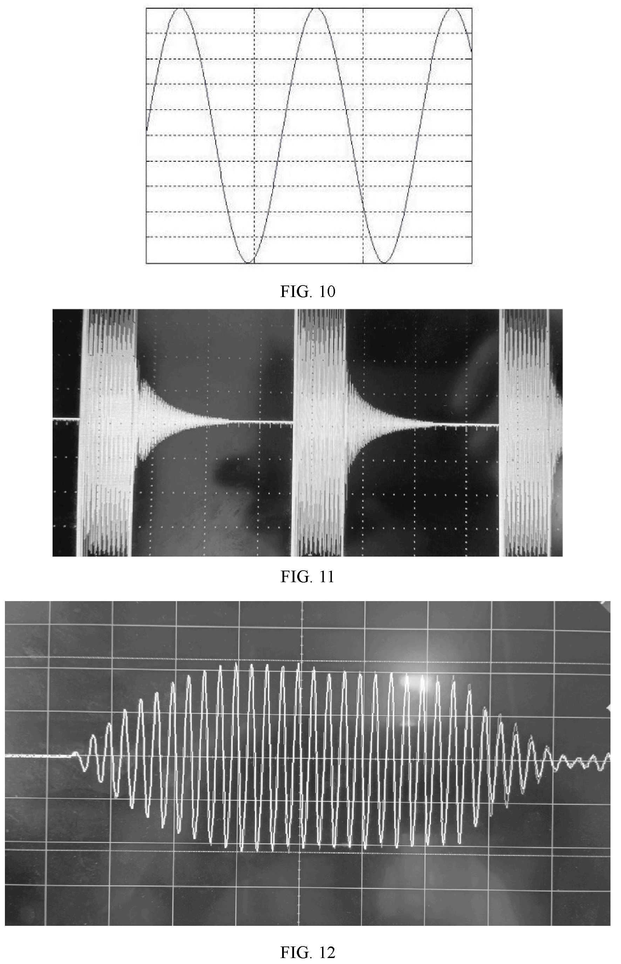

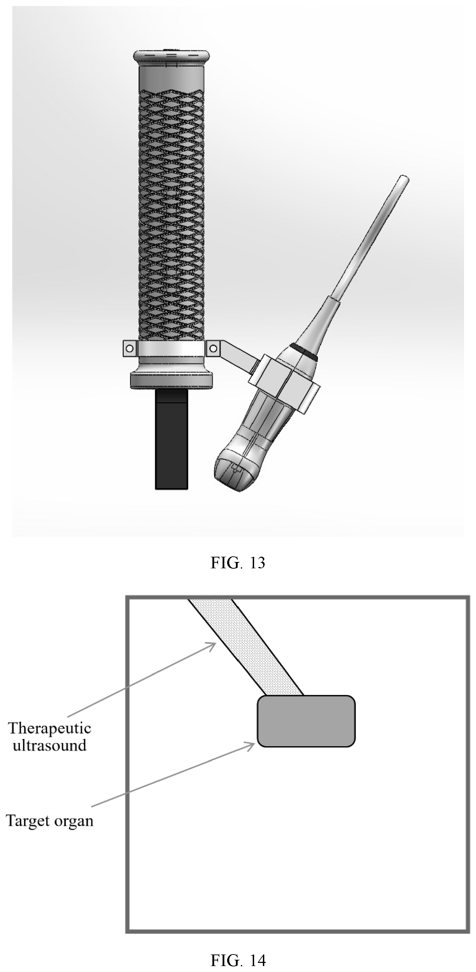

The ultrasonic waves output by the present device are in the form of a pulse-modulated sine wave. A schematic diagram of the sine wave is shown in FIG. 10, wherein the horizontal axis represents time and the vertical axis represents output intensity. In the present device, the output frequency ranges from 20 to 50 kHz and the output intensity (*i.e.*, ultrasound intensity) ranges from 0.1 to 1.6 W/cm². A schematic diagram of the sine wave modulation is shown in FIG. 11. In the modulated waveform, the emission duration corresponds to the pulse width; the interval from one emission start point to the next emission start point corresponds to the period; and the duty cycle is defined as the pulse width divided by the period. In the present device, the pulse width ranges from 10 to 1000 ms and the period ranges from 20 to 2000 ms, corresponding to a duty cycle of 1/2 to 1/200. The total treatment duration for the present device ranges from 60 to 600 s. The ultrasonic waves output by the present device are further processed using a smooth-transition algorithm, as shown in FIG. 12. For each pulse output, the intensity gradually increases from a low level to the target output intensity, which is maintained for one pulse width. At the end of the pulse, the intensity gradually decreases. Such an output mode improves patient comfort and tolerability, while also enhancing operational stability of the device and reducing the likelihood of damage. It more closely mimics the ultrasonic emissions of bats, thereby providing a more realistic biomimetic effect. Parameters such as output amplitude and duty cycle may be edited to generate treatment sequences according to therapeutic requirements, making the device more intelligent and user-friendly. If additional characteristics of ultrasonic emissions of bats are identified in the future, the biomimetic effect may be further refined by modifying the treatment sequences.
- The positioning module is an optional module used for locating a treatment area. When precise positioning is required, a miniature B-mode ultrasound probe may be typically employed. The B-mode ultrasound probe operates in conjunction with a B-mode ultrasound main unit to visualize internal organs and tissues. By securing the probe of the positioning module to the treatment head of the present disclosure, the internal organs and tissues can be visualized on the image of the positioning module, and the acoustic pathway of therapeutic ultrasound can be determined based on their relative positions. According to therapeutic requirements, appropriate acoustic pathways and target organs are selected for immunotherapy. The mounting configuration of the positioning module and the treatment head is shown in FIG. 13. A schematic diagram of the therapeutic ultrasound and the target organ on the image from the positioning module is shown in FIG. 14.

### Example 2: Verification of anti-tumor effects of ultrasound therapy system

A mouse liver tumor model was established to verify that low-frequency and low-intensity ultrasound applied to the immune system in a body can stimulate immune responses in the body, thereby exerting anti-viral and anti-tumor effects. The experimental groups are shown in Table 1.

**Table 1: Experimental groups**

| Group | Number of animals | Description |
|---|---|---|
| Group A: Pre-inoculation treatment | 10 mice | Treatment was administered before tumor inoculation. Treatment was performed three times for three consecutive days, followed by establishment of a tumor model. |
| Group B: Post-inoculation treatment | 7 mice | Treatment was administered after tumor inoculation when the tumor was visible to the naked eye. Treatment was performed three times for three consecutive days. |
| Group C: Normal control | 9 mice | Tumor inoculation was performed without any treatment. |

| | | |
|---|---|---|
| Note: Tumor inoculation was initiated simultaneously in all experimental mice, and tissue samples were collected for analysis and treatment at corresponding time points. The experiment was terminated when the tumor volume exceeded 1,500 mm³ or when the maximum tumor diameter was greater than 15 mm. Treatment site: Spleen of the mice to be treated | | |

### Treatment method:

The dedicated ultrasound therapy system developed according to the present disclosure was used to perform ultrasound treatment on the spleens of the experimental mice. A rectangular tool head suitable for spleen treatment was used in this experiment. Prior to the experiment, the mice were shaved, and the treatment site (spleen) was localized using B-mode ultrasound imaging and marked on the skin surface. The mice were anesthetized before ultrasound treatment.

### Treatment parameters:

- Ultrasound frequency: 33 kHz
- Ultrasound intensity: 0.2 W/cm²
- Treatment duration: 180 s
- Modulation pulse width: 100 ms
- Modulation period: 400 ms

### Indicator measurements

1. At the end of the experiment, tumor tissues are collected and photographed, followed by histopathological examination using H&E staining.
2. Serum levels of IFN-α, IFN-β, IFN-γ, and IL-15 were measured.
3. CD4+ T cells, CD8+ T cells, the ratio of CD8/CD4 T cells, and CD40+ immune cells in the blood were analyzed by flow cytometry.

### Experimental results

### 1. Relationship between tumor size and ultrasound treatment

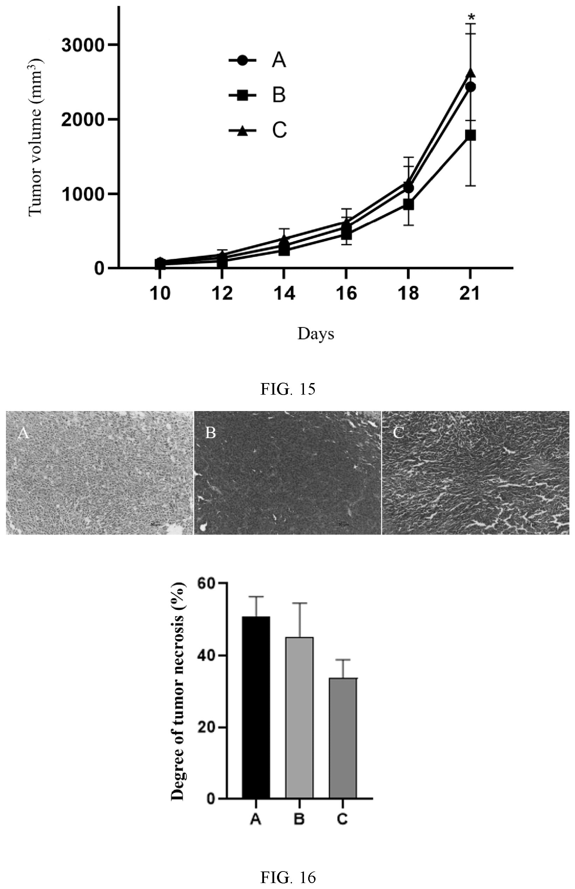

As shown in FIG. 15, the overall tumor volume followed the pattern: Group C > Group A > Group B. Compared with the normal model control group (Group C), tumor growth in Group A (ultrasound treatment followed by model establishment) was inhibited, but the inhibitory effect was not significant, indicating no statistical significance; tumor growth in Group B (model establishment followed by ultrasound treatment) was significantly inhibited, indicating statistical significance (*P* ≤ 0.05).

### 2. Histopathological analysis of tumors

As shown in FIG. 16, the degree of tumor necrosis followed the pattern: Group A > Group B > Group C. Compared with the normal model control group (Group C), both Group A (ultrasound treatment followed by model establishment) and Group B (model establishment followed by ultrasound treatment) exhibited significantly increased tumor necrosis, and Group A exhibited higher tumor necrosis than Group B. However, no statistically significant differences were observed among the groups.

### 3. Relationship between cytokine levels and ultrasound treatment

**Table 2: Statistical analysis of cytokines in the blood of mice**

| No. | Item | A | B | C |
|---|---|---|---|---|
| 1 | IFN-α | 106.73 ± 28.6 | 128.69 ± 20.72^{b} | 90.46 ± 20.83 |
| 2 | IFN-β | 213.80 ± 69.89 | 258.28 ± 61.04^{a} | 190.59 ± 43.73 |
| 3 | IFN-γ | 97.11 ± 32.28 | 110.54 ± 22.93^{a} | 82.13 ± 11.98 |
| 4 | IL-15 | 227.03 ± 22.1^{a} | 246.73 ± 34.8^{b} | 173.72 ± 39.2 |

| | | | | |
|---|---|---|---|---|
| Note: a: *P* < 0.05 vs. C; b: *P* < 0.01 vs. C. | | | | |

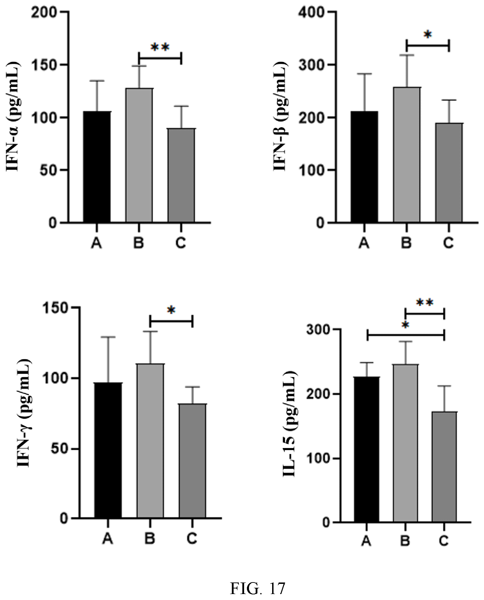

Experimental results: Analysis of cytokines in the blood of mice by ELISA revealed the following findings (Table 2 and FIG. 17):
No. 1: The serum IFN-α levels in experimental groups A, B, and C followed the pattern: Group B > Group A > Group C, with values of 106.73 ± 28.6 pg/mL, 128.69 ± 20.72 pg/mL, and 90.46 ± 20.83 pg/mL, respectively. Compared with the normal model control group (Group C), Group A exhibited increased serum IFN-α levels, indicating no statistical significance; Group B exhibited a significant increase in serum IFN-α levels, indicating statistical significance (*P* ≤ 0.01).
No. 2: The serum IFN-β levels in experimental groups A, B, and C followed the pattern: Group B > Group A > Group C, with values of 213.80 ± 69.89 pg/mL, 258.28 ± 61.04 pg/mL, and 190.59 ± 43.73 pg/mL, respectively. Compared with the normal model control group (Group C), Group A exhibited a slight increase in serum IFN-β levels, indicating no statistical significance; Group B exhibited a significant increase in serum IFN-β levels, indicating statistical significance (*P* ≤ 0.05).
No. 3: The serum IFN-γ levels in experimental groups A, B, and C followed the pattern: Group B > Group A > Group C, with values of 97.11 ± 32.28 pg/mL, 110.54 ± 22.93 pg/mL, and 82.13 ± 11.98 pg/mL, respectively. Compared with the normal model control group (Group C), Group A exhibited a slight increase in serum IFN-γ levels, indicating no statistical significance; Group B exhibited a significant increase in serum IFN-γ levels, indicating statistical significance (*P* ≤ 0.05).
No. 4: The serum IL-5 levels in experimental groups A, B, and C followed the pattern: Group B > Group A > Group C, with values of 97.11 ± 32.28 pg/mL, 110.54 ± 22.93 pg/mL, and 82.13 ± 11.98 pg/mL, respectively. Compared with the normal model control group (Group C), both Group A and Group B exhibited significantly increased serum IL-5 levels, indicating statistical significance (*P* ≤ 0.05), and Group B exhibited higher IL-15 levels than Group A.

### 4. Relationship between immune cells and ultrasound treatment

**Table 3: Statistical analysis of immune cells in the blood of mice**

| No. | Item | A | B | C |
|---|---|---|---|---|
| 1 | CD4+ T (%) | 20.48 ± 8.05 | 26.41 ± 5.45^{a} | 15.31 ± 9.08 |
| 2 | CD8+ T (%) | 8.54 ± 2.97 | 8.43 ± 2.79 | 8.73 ± 2.12 |
| 3 | CD40+ immune cells (%) | 30.68 ± 4.67 | 28.91 ± 7.92 | 25.13 ± 5.13 |
| 4 | CD8/CD4 T (%) | 44.03 ± 14.35^{a} | 32.66 ± 10.31^{b} | 72.78 ± 41.28 |

| | | | | |
|---|---|---|---|---|
| Note: a: *P* < 0.05 vs. C; b: *P* < 0.01 vs. C. | | | | |

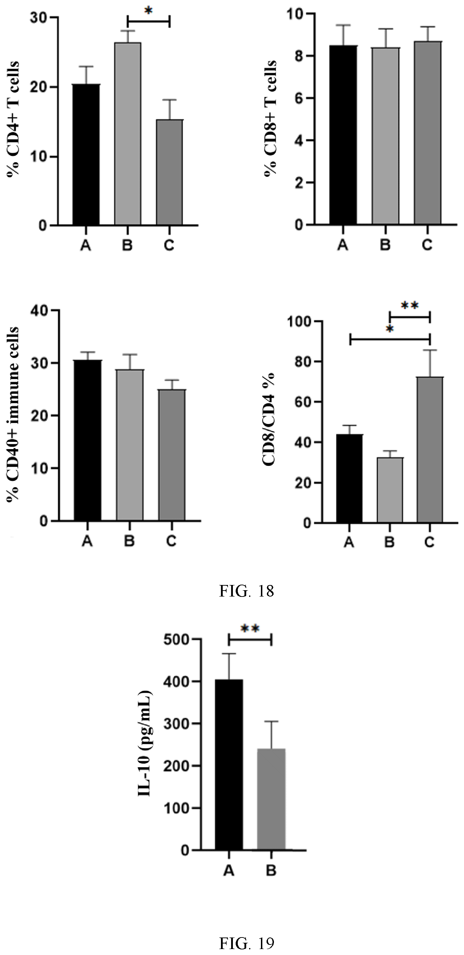

Experimental results: Analysis of the percentage of immune cells in the blood of mice by ELISA revealed the following findings (Table 3 and FIG. 18):
No. 1: The percentage of CD4+ T cells in the blood of mice in experimental groups A, B, and C followed the pattern: Group B > Group A > Group C, with values of 20.48 ± 8.05%, 26.41 ± 5.45%, and 15.31 ± 9.08%, respectively. Compared with the normal model control group (Group C), Group A exhibited an increase in the percentage of CD4+ T cells in the blood of mice, indicating no statistical significance; Group B exhibited a significant increase in the percentage of CD4+ T cells in the blood of mice, indicating statistical significance (*P* ≤ 0.05).
No. 2: The percentage of CD8+ T cells in the blood of mice in experimental groups A, B, and C showed no significant difference, with values of 8.54 ± 2.97%, 8.43 ± 2.79%, and 8.73 ± 2.12%, respectively. Compared with the normal model control group (Group C), neither Group A nor Group B exhibited changes in the percentage of CD8+ T cells in the blood of mice.
No. 3: The percentage of CD40+ immune cells in the blood of mice in experimental groups A, B, and C followed the pattern: Group A > Group B > Group C, with values of 30.68 ± 4.67%, 28.91 ± 7.92%, and 25.13 ± 5.13%, respectively. Compared with the normal model control group (Group C), both Group A and Group B exhibited a slight increase in the percentage of CD40+ immune cells in the blood of mice, indicating no statistical significance.
No. 4: The ratio of CD8/CD4 T cells in the blood of mice in experimental groups A, B, and C followed the pattern: Group C > Group A > Group B, with values of 44.03 ± 14.35%, 32.66 ± 10.31%, and 72.78 ± 41.28%, respectively. Compared with the normal model control group (Group C), both Group A and Group B exhibited a significant decrease in the ratio of CD8/CD4 T cells in the blood of mice, indicating statistical significance (*P* ≤ 0.05), and Group B exhibited a lower ratio than Group A.

### Example 3: Verification of ultrasound therapy system for prevention and amelioration of inflammatory aging

A model of 12-month-old rats was established to verify that low-frequency and low-intensity ultrasound applied to the immune system in a body can stimulate immune responses in the body, thereby preventing and ameliorating inflammatory aging, extending lifespan, and/or treating autoimmune diseases. The experimental groups are shown in Table 4.

**Table 4: Experimental groups**

| Group | Number of animals | Description |
|---|---|---|
| Group A: Treatment group | 9 rats | Treatment was performed once every other day for a total of five treatments. |
| Group B: Control group | 10 rats | Control group; no treatment was performed. |

### Treatment site:

### Spleen of the rats to be treated

### Treatment method:

The dedicated ultrasound therapy system developed according to the present disclosure was used to perform ultrasound treatment on the spleens of the experimental rats. A rectangular tool head suitable for spleen treatment was used in this experiment. Prior to the experiment, the rats were shaved, and the treatment site (spleen) was localized using B-mode ultrasound imaging and marked on the skin surface. The rats were anesthetized before ultrasound treatment.

### Treatment parameters:

- Ultrasound frequency: 50 kHz
- Ultrasound intensity: 0.2 W/cm²
- Treatment duration: 180 s
- Modulation pulse width: 100 ms
- Modulation period: 400 ms

### Indicator measurements

- At the end of the experiment, serum IL-10 levels in the rats were measured.

### Experimental results

- Relationship between IL-10 levels and ultrasound treatment

**Table 5: Statistical analysis of IL-10 levels in the blood of rats**

| No. | Item | A | B |
|---|---|---|---|
| 1 | IL-10 (pg/mL) | 405.01 ± 60.89^{a} | 241.22 ± 63.95 |

| | | | |
|---|---|---|---|
| Note: a: *P* < 0.01 vs. B. | | | |

Experimental results: Analysis of cytokines in the blood of rats by ELISA revealed the following findings (Table 5 and FIG. 19):
No. 1: The serum IL-10 levels in experimental groups A and B followed the pattern: Group A > Group B, with values of 405.01 ± 60.89 pg/mL and 241.22 ± 63.95 pg/mL, respectively. Compared with the control group (Group B), the serum IL-10 levels in Group A were significantly increased, indicating statistical significance (*P* ≤ 0.01).

IL-10 plays an important role in anti-inflammatory and immunoregulatory processes, and is closely associated with inflammatory aging. By maintaining IL-10 levels and function, the process of inflammatory aging may be slowed, thereby contributing to lifespan extension.

Although specific embodiments of the present disclosure have been described above, those skilled in the art should understand that these embodiments are merely illustrative, and various changes or modifications may be made to these embodiments without departing from the principles and essence of the present disclosure. Therefore, the scope of protection of the present disclosure shall be defined by the appended claims.

## Claims

1. An ultrasound therapy system, comprising a main unit and a treatment head; wherein
the main unit comprises the following modules:
(1) a signal module, configured to generate a fundamental frequency signal of ultrasound;
(2) a power supply module, configured to provide a constant DC voltage;
(3) an output module, configured to amplify the signal output;
(4) a matching module, configured to match the output signal with a piezoelectric module of the treatment head to achieve consistency in voltage and current phases;
the treatment head comprises the following modules:
(i) a piezoelectric module, configured to convert electrical energy into mechanical energy;
(ii) an amplitude transformer module, configured to output the vibration generated by the piezoelectric module;
the lengths of the piezoelectric module and the amplitude transformer module are each half of a wavelength;
the frequency range output by the ultrasound therapy system conforms to the frequency range of ultrasonic waves output by bats.

2. The ultrasound therapy system according to claim 1, wherein
the main unit further comprises: (5) a display module, configured to set parameters for ultrasound therapy, including frequency, sound intensity, duration, and/or duty cycle, and to provide functions for initiating and terminating ultrasound therapy;
the treatment head further comprises: (iii) a packaging module, configured to encapsulate and secure the piezoelectric module and the amplitude transformer module therein; and/or
the ultrasound therapy system further comprises a positioning module for locating a treatment area.

3. The ultrasound therapy system according to claim 1, wherein the ultrasonic waves output by the ultrasound therapy system are in the form of a sine wave; the frequency output by the ultrasound therapy system ranges from 20 to 50 kHz, with an output intensity ranging from 0.1 to 1.6 W/cm², a pulse width ranging from 10 to 1,000 ms, and a period ranging from 20 to 2,000 ms, corresponding to a duty cycle of 1/2 to 1/200;
preferably, the frequency output by the ultrasound therapy system ranges from 30 to 40 kHz, the output intensity ranges from 0.15 to 0.25 W/cm², the pulse width ranges from 50 to 200 ms, and the period ranges from 300 to 500 ms; or, the frequency output by the ultrasound therapy system ranges from 30 to 50 kHz, the output intensity ranges from 0.15 to 0.25 W/cm², the pulse width ranges from 50 to 200 ms, and the period ranges from 300 to 400 ms.
more preferably, the ultrasonic waves output by the ultrasound therapy system are further processed using a smooth-transition algorithm.

4. The ultrasound therapy system according to claim 2, wherein the ultrasound therapy system satisfies one or more of the following conditions:
the display module comprises a liquid crystal screen;
the signal module generates the fundamental frequency signal of ultrasound by means of DDS;
the output module amplifies the signal output by means of a MOS transistor;
the piezoelectric module comprises one or more piezoelectric ceramic wafers; and
the positioning module is a B-mode ultrasound probe, preferably a miniature B-mode ultrasound probe;
preferably, the positioning module is fixed on one side of the treatment head, both being located on the same plane and forming an angle of 0° to 90°, such that the acoustic pathway of therapeutic ultrasound is displayed on the B-mode ultrasound image;
more preferably, the angle is 10° to 80°, preferably 30° to 60°.

5. The ultrasound therapy system according to claim 1, wherein the piezoelectric module satisfies one or more of the following conditions:
the piezoelectric module comprises four piezoelectric ceramic wafers;
the piezoelectric ceramic wafers are P42 piezoelectric ceramics; and
the piezoelectric ceramic wafers are arranged with alternating positive and negative electrodes.

6. The ultrasound therapy system according to claim 2, wherein the packaging module comprises: a front cover plate, a rear cover plate, a housing, and a fastener, wherein the density of the material of the rear cover plate is greater than that of the front cover plate; preferably, the packaging module satisfies one or more of the following conditions:
the front cover plate is made of aluminum alloy;
the rear cover plate is made of stainless steel;
the housing is adapted for handheld operation, preferably in a cylindrical shape; and
the fastener is a set screw configured to securely connect the respective modules; the set screw is preferably provided with a preload.

7. The ultrasound therapy system according to claim 1, wherein the amplitude transformer module is in a rectangular shape, a circular shape, a spherical surface shape, a honeycomb shape, a needle shape, or a ball head shape; preferably, the amplitude transformer module satisfies one or more of the following conditions:
the output surface of the amplitude transformer module is wrapped with a silicone rubber film;
the transition between different diameters of the amplitude transformer module is smoothed by a tangent circular surface; and
the amplitude transformer module further comprises a flange for mounting the housing; preferably, the flange is positioned at a node.

8. The ultrasound therapy system according to any one of claims 1 to 7, wherein the ultrasound therapy system is used for regulating the following changes in the body:
(1) increasing the expression of interferons and/or interleukins; the interferon comprises IFN-α, IFN-β, and IFN-γ, and the interleukin comprises IL-10 and IL-15;
(2) promoting the proliferation of CD4+ T cells and/or CD40+ immune cells, and/or reducing the ratio of CD8/CD4.

9. A method of stimulating an immune response in a body using the ultrasound therapy system according to any one of claims 1 to 8, wherein ultrasonic stimulation is administered to a target site, preferably an immune organ such as the spleen or brain, comprising the following steps:
(1) bringing the ultrasound therapy system close to or in contact with the target site;
(2) adjusting parameters such that the ultrasound therapy system generates and emits ultrasonic waves;
(3) adjusting the propagation direction, output frequency, intensity, pulse width, period, and output duration of the ultrasonic waves according to the location and area of the target site;
preferably, the stimulation of the immune response in the body comprises anti-tumor therapy, anti-viral therapy, prevention and amelioration of inflammatory aging, lifespan extension, and/or treatment of autoimmune diseases.

10. The method according to claim 9, wherein the frequency output by the ultrasound therapy system ranges from 20 to 50 kHz, with an output intensity ranging from 0.1 to 1.6 W/cm², a pulse width ranging from 10 to 1000 ms, and a period ranging from 20 to 2000 ms, corresponding to a duty cycle of 1/2 to 1/200; and the duration of ultrasonic stimulation ranges from 60 to 600 s.

11. The method according to claim 9 or 10, wherein the frequency output by the ultrasound therapy system ranges from 30 to 40 kHz, with an output intensity ranging from 0.15 to 0.25 W/cm², a pulse width ranging from 50 to 200 ms, and a period ranging from 300 to 500 ms, corresponding to a duty cycle of 1/2 to 1/200; and the duration of ultrasonic stimulation ranges from 120 to 240 s; or, the frequency output by the ultrasound therapy system ranges from 30 to 50 kHz, with an output intensity ranging from 0.15 to 0.25 W/cm², a pulse width ranging from 50 to 200 ms, and a period ranging from 300 to 400 ms, corresponding to a duty cycle of 1/2 to 1/200; and the duration of ultrasonic stimulation ranges from 120 to 240 s.

12. The method according to claim 11, wherein the frequency output by the ultrasound therapy system is 33 kHz, the output intensity is 0.2 W/cm², the pulse width is 100 ms, the period is 400 ms, and the duration of ultrasonic stimulation is 180 s; or, the frequency output by the ultrasound therapy system is 50 kHz, the output intensity is 0.2 W/cm², the pulse width is 100 ms, the period is 400 ms, and the duration of ultrasonic stimulation is 180 s.

13. The method according to any one of claims 9 to 12, wherein ultrasonic stimulation is administered to the target site when the body has already developed a tumor, has already been infected with a virus, has already developed inflammation, has already aged, or has already developed an autoimmune disease.

14. Use of the ultrasound therapy system according to any one of claims 1 to 8 in the manufacture of a device for stimulating an immune response in a body;
preferably, the stimulation of the immune response in the body comprises anti-tumor therapy, anti-viral therapy, prevention and amelioration of inflammatory aging, lifespan extension, and/or treatment of autoimmune diseases.

15. The ultrasound therapy system according to any one of claims 1 to 8, wherein the ultrasound therapy system is used for stimulating an immune response in a body;
preferably, the stimulation of the immune response in the body comprises anti-tumor therapy, anti-viral therapy, prevention and amelioration of inflammatory aging, lifespan extension, and/or treatment of autoimmune diseases.
